# EUROPEAN PATENT SPECIFICATION

(11) **EP 4 013 351 B1**
(45) Date of publication and mention of the grant of the patent: **01.04.2026**
(21) Application number: 19941019.2
(22) Date of filing: 01.10.2019
(51) Int. Cl.: A61F 2/24, A61F 2/06, A61F 2/958

(54) **PROSTHETIC HEART VALVE ASSEMBLY**
HERZKLAPPENPROTHESENANORDNUNG
ENSEMBLE VALVULE CARDIAQUE PROTHÉTIQUE

(30) Priority: 13.08.2019 IN 201921032756
(43) Date of publication of application: 22.06.2022
(73) Proprietor: Meril Life Sciences Pvt Ltd, Gujarat, Vapi 396191 (IN)
(72) Inventor: MINOCHA, Pramod Kumar, Vapi, Gujarat 396191 (IN); KOTHWALA, Deveshkumar Mahendralal, Surat, Gujarat 395003 (IN); GHIYAD, Hitendra Arjanbhai, Vapi, Gujarat 396195 (IN)
(74) Representative: Patel, Binesh
(86) International application number: PCT/IN2019/050725
(87) International publication number: WO 2021/028933

(56) References cited:
- EP-A1- 0 901 776
- WO-A1-2018/085812
- WO-A1-2018/085812
- US-A1- 2007 288 081
- US-A1- 2017 065 415
- US-A1- 2019 209 320
- US-B2- 7 914 487
- US-B2- 8 808 370

## Description

### FIELD OF INVENTION

The present invention relates to a medical device, more specifically, the present invention relates to a prosthetic heart valve assembly.

### BACKGROUND

Prosthetic heart valves have been used since many years for the treatment of a heart valve disease. In the natural history of aortic stenosis (a heart valve disease), variable degrees of heart blockages may occur. The heart blockages may occur due to calcium deposits near native heart valves say, annulus of the native heart valve. The said calcium deposits may expand progressively from the LVOT (Left Ventricular Outflow Tract) to the arterioventricular conduction system of a patient. In order to overcome the aforesaid conditions, the prosthetic heart valves are deployed inside a patient's lumen at a target location (i.e. a diseased heart valve of the patient) in a crimped condition. The conventional prosthetic heart valves obstruct the LVOT (Left Ventricular Outflow Tract) and are unable to accurately fit at the target location. Further, during radial crimping and radial expansion of the conventional prosthetic heart valves, unequal distribution of radial forces occur which may lead to non-uniform expansion of the said valves.

The said conventional valves include large diameters due to which non-uniform expansion of the said valve takes place at the target location. The size of the conventional prosthetic heart valves may not be similar to the native diseased valve thereby, affecting the nearby walls of the intraventricular septum and/or nearby papillary muscles of the native valve. The aforesaid condition develops stress due to compression or contraction of muscles in such areas. The said conventional valves may affect the nearby position of the diseased heart valve viz. left ventricular outflow tract (LVOT) and coronary ostia during the deployment process. The said conventional valves excite the cardiac conduction system of the patient and induce a new left bundle branch block (LBBB) during the deployment process. Due to the aforesaid disadvantages of the conventional prosthetic heart valves, an external assisting device such as a pacemaker (temporary/permanent type) is used at times post the deployment process for pacing the heart and to maintain its rhythmic condition.

Of late, transcatheter aortic valve implantation (TAVI) or replacement (TAVR) procedure is employed for the deployment of prosthetic heart valves for intermediate risk patients with severe aortic stenosis. The TAVI procedures are performed to implant prosthetic heart valves with the help of a delivery catheter which may include a balloon, one or more stoppers, etc. In the TAVI procedure, a valve is mounted in a crimped position on a balloon located on the catheter. The balloon is deployed from the femoral artery to the target location of the patient. The valve mounted on the balloon is inflated by providing an inflation fluid(s) inside the balloon and expanding the said valve at the target location.

However, in the conventional delivery catheters, precise positioning of the prosthetic heart valves and fitment of the said valves pose a big challenge. The aforesaid conditions may lead to severe complications during and after the process of deployment. The severe complications may include, valve embolization and coronary occlusion. Moreover, the design and dimensions of the conventional delivery catheters are unable to maintain the design integrity and uniformity of the prosthetic heart valves during the deployment process. The aforesaid deviations may be caused by various reasons such as push-resistance due to limited to artery condition, size, occlusions or fluid non-uniformity during inflation of the prosthetic heart valves may create conduction disturbances leading to LBB which increases the probability of usage of temporary or permanent pacemaker device post the deployment process. The conventional delivery catheters may be unable to uniformly expand the prosthetic heart valves and may not maintain the outer low profile of the delivery catheter. Further, the said conventional catheters may not retain the linearity and center of gravity of the prosthetic heart valves at crimped stage prior to its deployment to the target location.

For instance, the patents EP2736457B1 and US9119716 disclose the one or more stoppers which include one or more slots. Due to these slots, the crimped prosthetic heart valve loses its exact positioning thereby affecting its linearity and hence, its center of gravity. The center of gravity is also affected when a high push force is applied during the deployment process which also affects the flexibility of the prosthetic heart valve. Moreover, in the said prior art, an inner shaft/tube is being passed through the said stoppers. When the stoppers are crimped, the slots are also crimped and the gap between the inner shaft and the slot of the stopper becomes non-uniform. The aforesaid condition results in uneven flow of the fluid and thereby changes the uniformity of its hydro dynamics. The conventional stoppers thus, provide non-uniform inflation of catheter which results in occlusions near the target location.

Further, US2017/065415A1 discloses a delivery apparatus for implanting a radially compressible and expandable prosthetic heart valve in a native heart valve of the heart includes a handle portion and an elongated shaft extending from and movable relative to the handle portion. The delivery apparatus includes a gear assembly that causes corresponding longitudinal motion of the elongated shaft relative to the handle portion. US8808370B2 discloses a valve loading apparatus provided for loading a crimped prosthetic valve into a lumen of a delivery system and a valve recoil adapter can counteract recoil of a compressed prosthetic valve, and maintain the valve at its desired crimp diameter. WO2018/085812A1 discloses a delivery apparatus having a steerable shaft and an adjustment mechanism. The adjustment mechanism is configured to increase and decrease tension in a pull wire to adjust the curvature of the steerable shaft. US2007/288081A1 discloses a stent delivery system includes a stent stopper that impedes the stent from slipping from the balloon. However, none of these applications disclose a delivery system and a prosthetic valve that mitigates requirement of external pacing device.

Therefore, a delivery catheter including a prosthetic heart valve which overcomes the drawbacks of the conventional systems is required to be devised.

### SUMMARY

The invention relates to prosthetic heart valve assembly according to independent claim 1. Optional features are provided in dependent claims. A prosthetic heart valve assembly includes a prosthetic heart valve mounted over a balloon. The prosthetic heart valve is deployment at a target location i.e. within a diseased native valve. The dimensions of the prosthetic heart valve mimic the anatomy of a native heart valve.

A delivery catheter utilized for deployment of the prosthetic heart valve. The delivery catheter includes an outer shaft. The outer shaft further includes an outer first end, an outer second end and a lumen.

The outer first end has a diameter greater than the outer second end. The outer shaft includes one or more layers namely, an inner layer, a middle layer and an outer layer.

An inner shaft is provided inside the lumen of the outer shaft. The inner shaft includes an inner first end and an inner second end. The inner shaft includes one or more layers namely, a first layer, an intermediate layer and a second layer.

A support tube is disposed on top of the inner second end of the inner shaft. The support tube includes one or more holes for passing an inflation fluid(s). The support tube is provided with the balloon on its outer surface.

A handle is provided at an end of the delivery catheter. One or stoppers are provided on the support tube and within the balloon. The one or more stoppers include a first portion and a second portion.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein. Moreover, those in the art will understand that the drawings are not to scale.
Fig. 1 depicts a prosthetic heart valve in accordance with an embodiment of the present invention.
Fig. 1a depicts a delivery catheter in accordance with an embodiment of the present invention.
Fig. 1b depicts an outer shaft of the delivery catheter in accordance with an embodiment of the present invention.
Fig. 1c depicts an inner shaft of the delivery catheter in accordance with an embodiment of the present invention.
Figs. 1d- 1e depict the one or more layers of the inner shaft in accordance with an embodiment of the present invention.
Fig. 1f depicts an expanded view of the components of the delivery catheter in accordance with an embodiment of the present invention.
Figs. 1g and 1h depict the perspective views of the balloon and the one or more stoppers in accordance with an embodiment of the present invention.
Figs. 1i - 1k depict the different embodiments of the one or stoppers in accordance with an embodiment of the present invention.
Fig. 2 illustrates a flow chart depicting the deployment procedure of a prosthetic heart valve in accordance with an embodiment of the present invention.

### DETAILED DESCRIPTION OF THE DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Particular embodiments of the present disclosure are described herein below with reference to the accompanying drawings, however, it is to be understood that the disclosed embodiments are merely examples of the disclosure, which may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

It should be noted that the words 'valve', 'heart valve', 'prosthetic heart valve' and 'prosthetic valve' have been interchangeably used in the present disclosure to refer to a prosthetic heart valve and hence, the meaning of all stated phrases should be interpreted alike.

In accordance with the present disclosure, a prosthetic heart valve along with a delivery catheter (collectively referred to as a 'prosthetic heart valve assembly') is disclosed. The prosthetic heart valve of the present invention is deployed with the help of the delivery catheter at a target location (such as within a diseased native valve). The prosthetic heart valve of the present invention is so dimensioned (i.e. includes a pre-defined height, diameter and surface area) that it mimics the natural anatomy of a native heart valve. Therefore, the prosthetic heart valve does not obstruct the natural anatomy and function of the heart which results in eliminating the requirement of an external assisting device such as, pacemakers post-surgery to maintain the blood circulation inside a patient's body. In an embodiment, the prosthetic heart valve of the present invention does not disrupt the LVOT provided in the human heart. The said prosthetic heart valve ensures that all the small gaps near the native heart valve are entirely covered which further helps in preventing paravalvular leakage.

The delivery catheter of the present invention provides easy and accurate deployment of the prosthetic heart valve. The delivery catheter of the present invention includes one or more components. The one or more components includes a balloon, an outer shaft, an inner shaft, a support tube, one or more stoppers etc. (described below in detail). The one or more stoppers provided in the delivery catheter ensure accurate positioning of the prosthetic heart valve during transfer of the said valve from a femoral point of the patient to the target location. The one or more stoppers of the present invention may have at least one hole for air displacement and/or uniform fluid entry inside the balloon lumen.

The delivery catheter and the prosthetic heart valve of the present invention do not affect, compress and/or hamper the nearby muscles of the target location and the left ventricular outflow tract (LVOT). Further, the delivery catheter and the prosthetic heart valve of the present invention do not obstruct coronary ostia therefore, no external temporary/permanent pacing device (pacemaker) is required.

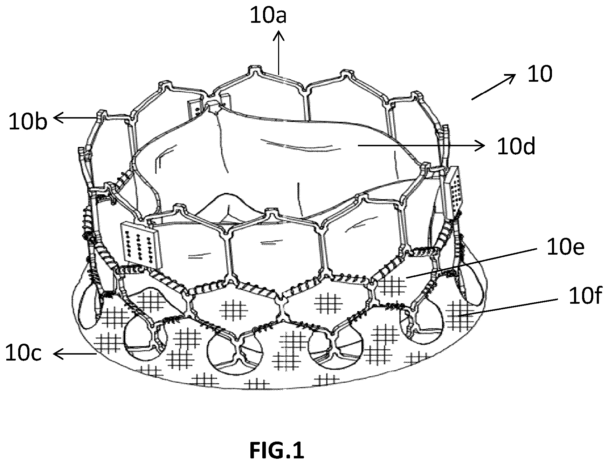

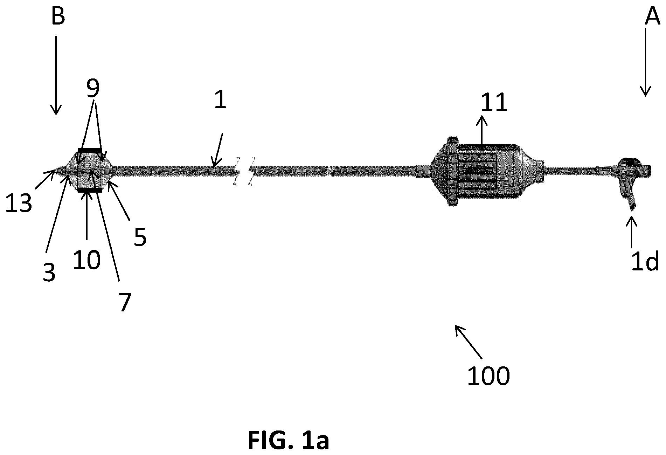

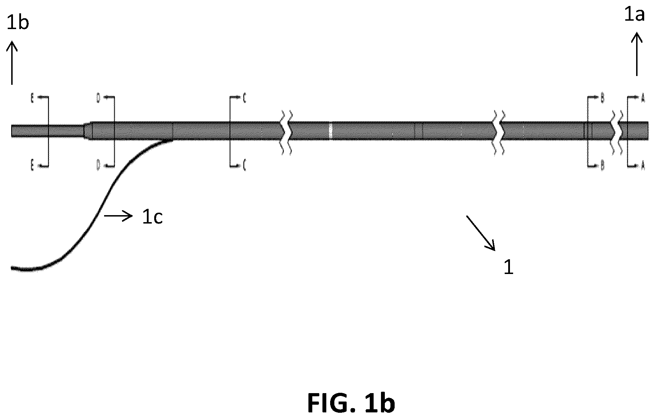

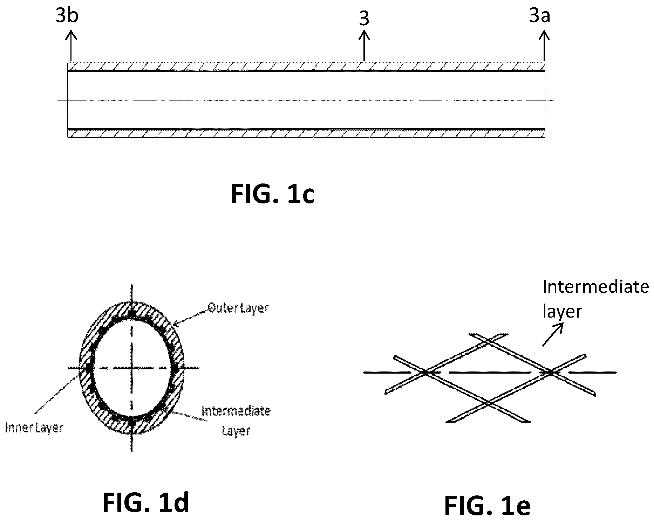

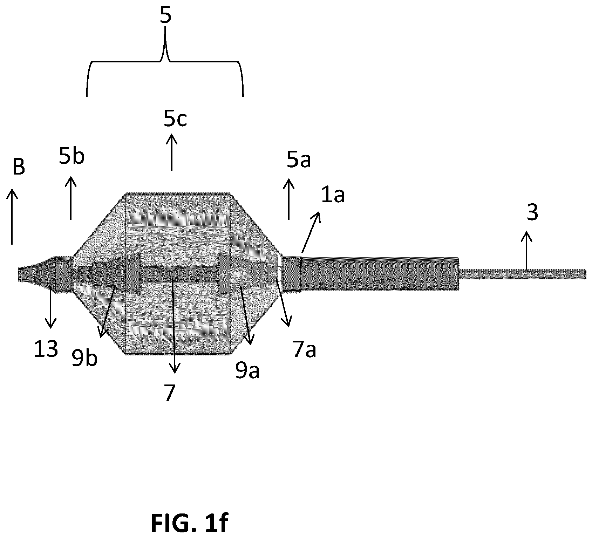

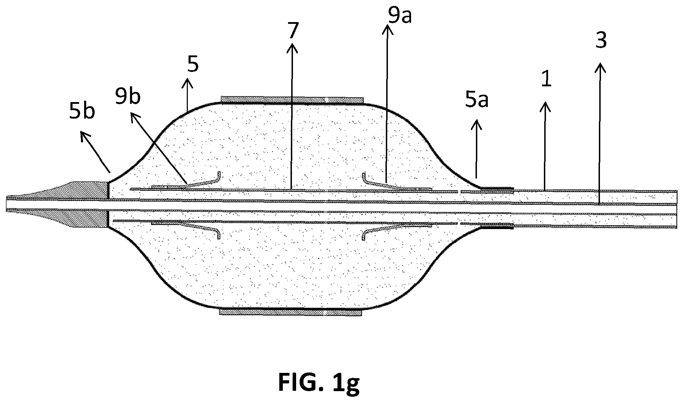

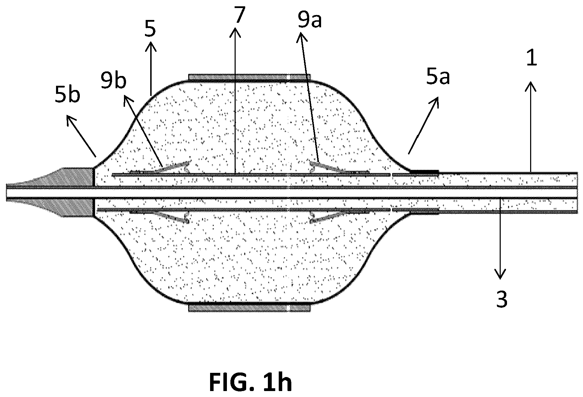

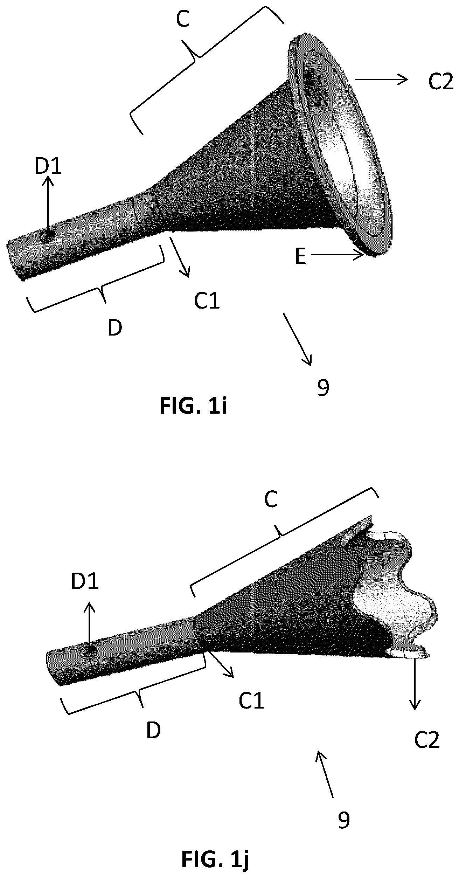

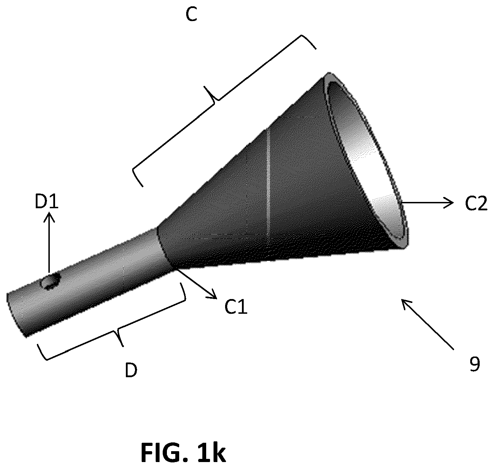

Now referring specifically to the drawings, Fig. 1 depicts a prosthetic heart valve 10 of the present invention. The prosthetic heart valve 10 is deployed within a diseased native valve i.e. a target location. The prosthetic heart valve 10 may be a balloon-expandable valve, that is, the prosthetic heart valve 10 is expanded by placing the said valve on a balloon. In an embodiment, the prosthetic heart valve 10 is deployed at the target location in a crimped state. The prosthetic heart valve 10 may be crimped on a balloon provided on a delivery catheter prior to implantation at the target location. The crimped profile of the prosthetic heart valve 10 may range from 6.8- 8.0 mm, more preferably 7.0- 7.6 mm. In an embodiment, the crimped profile of the prosthetic heart valve 10 is 7.3mm.

The prosthetic heart valve 10 is formed using predefined dimensions. The predefined dimensions of the prosthetic heart valve 10 may include without limitation, a surface area, a diameter and a height. In an embodiment, the predefined dimensions of the prosthetic heart valve 10 are selected from progressively increasing ranges. Due to the aforesaid ranges of progressive increase in the dimensions (surface area, diameter and height) of the prosthetic heart valve 10, the said valve 10 may be efficiently implanted in the native heart valves having annulus in the range of 260 mm² to 850 mm².

The progressively increasing range of surface area of the prosthetic heart valve 10 may be from 280mm²- 900mm². The progressive increase in surface area of the prosthetic heart valve 10 ranges between 30 mm² to 90 mm².

The progressively increasing range of diameter of the prosthetic heart valve 10 is from 18mm to 33.5mm. The progressive increase in diameter of the prosthetic heart valve 10 ranges from 1mm- 2mm.

The progressively increasing range of height of the prosthetic heart valve 10 is in the range of 14mm to 28mm, more preferably 17- 25 mm. The progressive increase in height of the prosthetic heart valve 10 ranges from 0.2mm-1mm.

The progressively increasing dimensions of the prosthetic heart valve 10 are selected in a way that the prosthetic heart valve 10 mimics the natural anatomy of the diseased native valve. The prosthetic heart valve 10 selected from progressively increasing dimensions do not affect the nearby walls of intraventricular septum and/or nearby papillary muscle of the native heart valve during the deployment of the said valve 10. The prosthetic heart valve 10 selected from progressively increasing dimensions does not develop any stress due to the compression or contraction of the muscles in the intraventricular septum and/or nearby papillary muscle areas of the native heart valve.

The prosthetic heart valve 10 of the present invention does not affect the position of the diseased native heart valve and does not obstruct the LVOT and the coronary ostia of the patient during the deployment process. The progressively increasing dimensions of the prosthetic heart valve 10 ensure that during the deployment process the valve does not excite the cardiac conduction system of the patient and does not induce a new LBBB. Thus, the prosthetic heart valve 10 eliminates the possibility of a temporary and/or permanent pacemaker (PPM) post its implantation. In an embodiment, the clinical results after deploying the prosthetic heart valve 10 shows that the temporary/permanent pacing devices (pacemaker) required post implantation of the prosthetic heart valve 10 of the present invention are found below 2% as compared to the conventional transcatheter aortic valve implantation systems in which the temporary/permanent pacing devices (pacemaker) required post implantation is 12% to 15%.

A table depicting the progressively increasing dimensions of the successive prosthetic heart valves 10 is provided below:

**Table 1**

| **S. No.** | **Valve Area (mm²)** | **Valve Diameter (mm)** | **Valve Height (mm)** |
|---|---|---|---|
| 1 | 314 | 20 | 17.35 |
| 2 | 363 | 21.5 | 17.60 |
| 3 | 415 | 23 | 17.85 |
| 4 | 471 | 24.5 | 18.35 |
| 5 | 531 | 26 | 18.85 |
| 6 | 594 | 27.5 | 19.60 |
| 7 | 661 | 29 | 20.35 |
| 8 | 731 | 30.5 | 21.15 |
| 9 | 804 | 32 | 22.0 |
| 10 | 881 | 33.5 | 22.8 |

The prosthetic heart valve 10 may include without limitation, a frame 10a, an outflow end 10b, an inflow end 10c, at least one leaflet 10d, an inner skirt 10e and an outer skirt 10f.

An example showing the advantages of using a prosthetic heart valve 10 that mimics a native heart valve is depicted below:
**Example 1:** A patient having aortic annulus area of 545mm² requires implantation of a transcatheter aortic valve. Based on the conventional sizes of the prosthetic heart valves available, the physician selects a heart valve of either 649mm² or 660mm² surface area (Ref: Eur Heart J Cardiovasc Imaging. 2016 Sep; 17(9): 1054-1062) with a diameter of 28mm and a frame height of 20.35mm. However, as per the sizing parameters of the prosthetic heart valve 10 of the present invention, the physician may select a prosthetic valve which is closer to the available area of 594mm².

The excess area occupied by a conventional prosthetic heart valve is 104 mm² to 110mm² (which is higher by 19.08% to 20.18% of the required area). As opposed to the higher area occupied by the conventional prosthetic heart valve, the prosthetic heart valve 10 of the present invention exceeds the area by only 8.80%. Thus, the conventional valves are likely to hamper the native heart blood circulation in a patient at large. Therefore, the nearby walls of the intraventricular septum and/or nearby papillary muscles of the diseased native heart valve are disturbed further affecting the electric impulses.

The conventional valves may pose several other problems due to obstruction of blood flow which in turn, may affect the functioning of an adjacent valve, say mitral valve. Further, as an excessive area of the target location is covered the chances of implanting a permanent and/or a temporary pacemaker increases in order to regulate the normal function of the prosthetic heart valve. The aforesaid problems are minimized by implanting the prosthetic heart valve 10 of the present invention which mimics the natural anatomy of the native heart valve. Therefore, the need of implanting a permanent and/or a temporary pacemaker is eliminated which in turn eliminates the aforesaid problems.

Fig. 1a depicts a delivery catheter 100 of the present invention. The delivery catheter 100 is utilized for deploying a prosthetic heart valve (say, the prosthetic heart valve 10) within a diseased native valve i.e. a target location. The delivery catheter 100 provides easy and accurate deployment of the prosthetic heart valve 10 at the target location. The length of the delivery catheter 100 may be in the range of 1300- 1700mm, more preferably 1450-1550mm. In an embodiment, the length of the delivery catheter 100 is 1500 mm.

The working length or the usable length of the delivery catheter 100 may be in the range of 1000- 1400mm, more preferably 1150- 1250mm. In an embodiment, the working length or the usable length of the delivery catheter 100 is 1200 mm.

The delivery catheter 100 includes a primary end (marked as 'A' in Fig. 1a) and a secondary end (marked as 'B' in Fig. 1a). The delivery catheter 100 further includes one or more components. The one or more components may include without limitation, an outer shaft/outer lumen/outer sheath 1, an inner shaft or inner lumen 3, a balloon 5, a support tube 7, one or more stoppers 9, a handle 11 and a soft tip 13.

The outer shaft 1 acts as a top layer for the delivery catheter 100. The outer shaft 1 includes an outer first end 1a, an outer second end 1b, a pull wire 1c (as depicted in Fig. 1b) and a hub 1d (depicted in Fig. 1a). In an embodiment, the outer first end 1a is provided near a proximal end of the balloon 5 of the delivery catheter 100 and the outer second end 1b is provided near the hub 11. The total length of the outer shaft 1 may be in a range of 1100-1600mm, more preferably 1300- 1400mm. In an embodiment, the diameter of the outer first end 1a may vary from the outer second end 1b of the outer shaft 1.

The outer first end 1a of the outer shaft 1 may include an outer diameter in the range of 2.5- 6.5mm, more preferably 4.0mm to 5.0mm and an inner diameter in the range of 0.5-4.5mm, more preferably 2.0- 3.0mm. The outer second end 1b of the outer shaft 1 may include an outer diameter in the range of 1.5- 5.5mm, more preferably 3.0- 4.0mm and an inner diameter is the in range of 0.5- 4.5mm, more preferably 2.0- 3.0mm.

In an embodiment, the hub 1d is utilized to pass an inflation fluid(s) to expand the balloon 5.

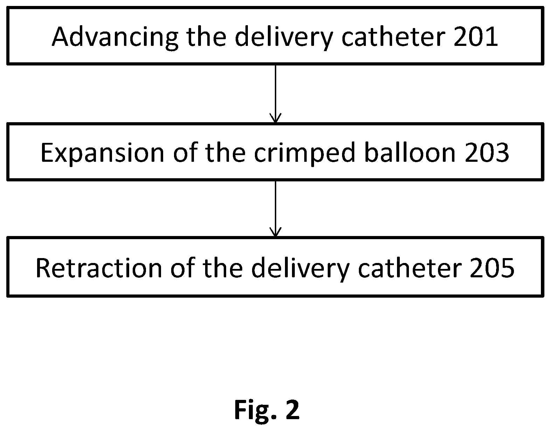

The outer shaft 1 may be in the form of a hollow tube including a lumen (not shown). In an embodiment, the outer shaft 1 acts as a steering mechanism having a pull and release wire mechanism which is carried out with the pull wire 1c (explained in Fig. 2). The said steering mechanism provides suitable flexion during the navigation/implantation of the prosthetic heart valve 10 at the target location.

The outer shaft 1 may be a single layer shaft or a multi-layer shaft. In an embodiment, the outer shaft 1 is a multi-layer shaft. The outer shaft 1 may include without limitation, an inner layer, a middle layer and an outer layer (not shown). The inner layer of the outer shaft 1 may be made of without limitation polytetrafluoroethylene (PTFE), nylon or any other polymer.

The inner layer of the outer shaft 1 may be covered with a middle layer. In an embodiment, the middle layer of the outer shaft 1 is braided over the inner layer to provide flexibility at different locations during the deployment procedure. The braiding of the middle layer of the outer shaft 1 may be performed using, without limitation, stainless steel, nitinol or cobalt chromium flat or round wires. The process of braiding is performed using multiple metal wires. The multiple metal wires may be in the range of 4 wires to 32 wires, more preferably 8 wires to 24 wires.

In an embodiment, the outer first end 1a of the outer shaft 1 is braided using any of the aforesaid wires. The length of the outer first end 1a of the outer shaft 1 may be in the range of 950- 1300mm, more preferably 1100- 1150mm. The braiding is performed at the outer first end 1a of the outer shaft 1 to provide sufficient strength, flexibility and kink resistance properties to the outer shaft 1. Moreover, the different grades of polymers are welded together in the outer shaft 1 at specific locations to help in steering the outer shaft 1.

In an embodiment, the outer second end 1b of the outer shaft 1 is not braided. The length of the outer second end 1b of the outer shaft 1 may be in the range of 05-200mm, more preferably 50-120mm.

In an embodiment, the pull wire 1c utilized during the steerable mechanism is braided using multiple metal wires of the middle layer of the outer shaft 1. The pull wire 1c may be a monofilament wire or a multifilament wire. In an embodiment, the pull wire 1c is a monofilament wire. The pull wire 1c may also include a pull ring (not shown) which helps in providing steerable mechanism to the outer shaft 1. The pull wire 1c coupled with the pull ring is provided on the outer first end 1a of the outer shaft 1. The length of the pull wire 1c may be in a range of 1000- 1600mm, more preferably 1200- 1400mm.

The middle layer of the outer shaft 1 may be further covered with the outer layer of the outer shaft 1 to maintain the flexibility of the outer shaft 1 at different locations during the deployment procedure. The outer layer of the outer shaft 1 may be made of without limitation, a variety of polymeric materials such as nylon, polytetrafluoroethylene (PTFE), pebax, polyamide, polyether ether ketone (PEEK), silicone and other derivatives. In an embodiment, the outer shaft 1 is made of pebax and polyamide resin. The grades of pebax utilized for the formation of the outer shaft 1 include pebax 2533, pebax 3533, pebax 4033, pebax 5533, pebax 6333, pebax 7033, pebax 7233, and pebax 7433. The outer shaft 1 is made from pebax as the said material is biocompatible. Pebax offers efficient mechanical properties such as, light weight, higher flexibility, excellent physical properties and chemical resistance. Other materials with similar characteristics may also be used for the formation of the outer layer of the outer shaft 1.

The inner shaft 3 is provided inside the lumen of the outer shaft 1 (as depicted in Fig. 1c). The inner shaft 3 may include an inner first end 3a and an inner second end 3b. In an embodiment, the inner first end 3a is provided near the primary end 'A' of the delivery catheter 100 and the inner second end 3b is provided near the secondary end 'B' of the delivery catheter 100. The inner shaft 3 is provided inside the lumen of the outer shaft 1 from the primary end 'A' of the delivery catheter till the outer second end 1b of the outer shaft 1. From the outer first end 1a of the outer shaft 1, the inner shaft 3 enters inside the balloon 5 through a lumen of the support tube 7 and extends till the secondary end 'B' of the delivery catheter 100 wherein the inner second end 3b of the inner shaft 3 is provided. The inner shaft 3 also includes a lumen (not shown). The said lumen of the inner shaft 3 acts as a passage for a guide wire. The guide wire may be any guide wire known in the art.

The inner shaft 3 may be a single layer shaft or a multi-layer shaft. In an embodiment, the inner shaft 3 is a multi-layer shaft. The inner shaft 3 may include without limitation, a first layer, an intermediate layer and a second layer (as depicted in Fig. 1d). The first layer of the inner shaft 3 acts a base layer for the inner shaft 3. The first layer of the inner shaft 3 may be made of without limitation, nylon, PTFE, pebax, polyimide, PEEK, silicone etc. In an embodiment, the first layer of the inner shaft 3 is made of polyimide which provides smooth surface for less friction at guide wire movements.

The first layer of the inner shaft 3 is covered with an intermediate layer. In an embodiment, the intermediate layer of the inner shaft 3 has a braided configuration (as depicted in Fig. 1e). The intermediate layer may be braided using a round or a flat metal wire. The round or flat metal wires may be made of without limitation, nitinol, cobalt chromium or stainless steel (SS) material. In an embodiment, the braiding is done with stainless steel wire(s). The braiding may be performed at a braiding angle which may be in the range of 60° to 120°, more preferably 80° to 100°.

The intermediate layer of the inner shaft 3 is covered with a second layer. In an embodiment, the second layer of the inner shaft 3 may act as a cover for the inner shaft 3. The second layer of the inner shaft 3 may be made of without limitation, nylon, PTFE, polyimide, pebax, PEEK, silicone and other derivatives. In an embodiment, the second layer of the inner shaft 3 is made of polyimide which provides flexibility to the first layer of the inner shaft 3.

The length of the inner shaft 3 may be in the range of 1200- 1800mm, more preferably 1400- 1600mm. The inner shaft 3 may include an outer diameter in the range of 0.1- 2.5mm, more preferably between 1.0- 1.5mm and an inner diameter in the range of 0.1- 2.0mm, more preferably between 0.6- 1.2mm.

The balloon 5 is provided towards the secondary end 'B' of the delivery catheter 100. The balloon 5 includes a proximal end 5a, a distal end 5b and a middle portion 5c (as depicted in Fig. 1f). In an embodiment, the proximal end 5a of the balloon 5 is coupled with the outer second end 1b of the outer shaft 1 and the distal end 5b of the balloon 5 is coupled to the soft tip 13. The balloon 5 may be a single layer member or a multi-layer member. In an embodiment, the balloon 5 is a single layer member. In an embodiment, the middle portion 5c of the balloon 5 is hollow and cylindrical in shape. The proximal end 5a and the distal end 5b of the balloon 5 are conical in shape.

The balloon 5 may be made of a variety of polymeric materials which may include without limitation, nylon, pebax, polyethylene terephthalate (PET), polyamide, polyurethanes, polyvinyl chloride, polyethylene, etc. In an embodiment, the balloon 5 is made of without limitation, polyamide resins.

The balloon 5 may be utilized for expansion of the prosthetic heart valve 10. In an embodiment, the prosthetic heart valve 10 is placed over the balloon 5. Hence, when the balloon 5 expands, the prosthetic heart valve 10 also expands. In an embodiment, the balloon 5 along with the prosthetic heart valve 10 is deployed in a crimped state and is inflated once the balloon 5 along with the prosthetic heart valve 10 reaches the target location. In an embodiment, once the balloon 5 along with the prosthetic heart valve 10 is completely expanded at the target location, the balloon 5 is retracted from the target location. The inflation of the balloon 5 along with the prosthetic heart valve 10 is performed with the help of an inflation fluid(s). The inflation fluid(s) may include without limitation, saline solution. In an embodiment, the amount of inflation fluid required to fill the balloon 5 is dependent on the diameter of the balloon 5. The volume of the inflation fluid(s) filled inside the balloon 5 may range from 5 to 100 ml and more preferably, 15 to 60 ml.

The proximal and distal ends (5a and 5b) of the balloon 5 may include a lumen which includes the support tube 7 (described below).

In an embodiment, the balloon 5 includes at least two markers (not shown). The at least two markers may be made of a radiopaque material. The at least two markers help in locating the prosthetic heart valve 10 inside a patient's aortic orifice lumen during the deployment procedure. The at least two markers are provided within a working length of the balloon 5 (i.e. within the portion of the balloon 5). The working length of the balloon 5 may be in the range of 15- 45mm, more preferably 25- 45mm. In an embodiment, the working length of the balloon 5 is 35mm.

The balloon 5 includes an outer diameter in range of 16- 36mm, more preferably from 20- 32 mm. In an embodiment, the outer diameter of the balloon 5 is dependent on the diameter of the prosthetic heart valve 10 in order to provide uniform expansion to the prosthetic heart valve 10.

The support tube 7 is provided inside the lumen of the balloon 5 (as depicted in Fig. 1f) and is disposed on top of the inner second end 3b of the inner shaft 3. The support tube 7 includes a lumen which supports the balloon 5 during the expansion and deflation of the prosthetic heart valve 10. The said lumen also helps to deliver the inflation fluid inside the balloon 5 in order to expand the balloon 5 which in turn expands the prosthetic heart valve 10. The support tube 7 further includes a distal opening and a proximal opening (not shown). In an embodiment, the proximal opening is provided near the proximal end 5a of the balloon 5 and the distal opening is provided near the distal end 5b of the balloon 5.

The support tube 7 may include one or more holes 7a near the distal and/or the proximal opening. In an embodiment, the one or more holes are provided near the proximal opening of the support tube 7. The distal opening and the one or more holes 7a provided in the proximal opening of the support tube 7 act as a passage for the inflation fluid(s). In an embodiment, the inflation fluid(s) enters the balloon 5 via the support tube 7. The distal opening and the one or more holes 7a provided in the proximal opening of the support tube 7 ensures uniform expansion of the balloon 5 which in turn ensures uniform expansion of the prosthetic heart valve 10 when deployed at the target location.

The support tube 7 may be made of any polymeric material. The polymeric material may include without limitation, nylon, PTFE, pebax, PEEK, silicone and other derivatives. In an embodiment, the support tube 7 is made of polyamide. The length of the support tube 7 may be in the rage of 40- 100mm, more preferably 50- 80mm. In an embodiment, the length of the support tube 7 along with the one or more stoppers 9 is 65-80mm. The length of the support tube depends on the length of the balloon 5.

The support tube 7 also includes an outer diameter and an inner diameter. The outer diameter of the support tube 7 may be in the range of 0.5- 4.5mm, more preferably 2.0- 3.0mm. The inner diameter of the support tube 7 may be in the range of 0.1- 4.0mm, more preferably 1.5- 2.5mm.

The one or more stoppers 9 are provided inside the balloon 5 at the proximal and distal end (5a and 5b) of the balloon 5 and are mounted on an outer surface (not shown) of the support tube 7. In an embodiment, the one or more stoppers 9 include two stoppers- a proximal stopper 9a and a distal stopper 9b (as depicted in Fig. 1f, 1g and 1h). The proximal stopper 9a is provided at the proximal end 5a of the balloon 5. The distal stopper 9b is provided at the distal end 5b of the balloon 5. The distance between the proximal stopper 9a and the distal stopper 9b when mounted on the outer surface of the support tube 7 may be in the range of 15- 50mm, more preferably 18- 40mm.

The one or more stoppers 9 may be made of without limitation, medical grade polyurethane, silicone, polyimide, nylon, PTFE and other polymer combinations. In an embodiment, the one or more stoppers 9 are made of polyurethane.

The one or more stoppers 9 may include a first portion (marked as 'C' in Figs. 1i-1k) and a second portion (marked as 'D' in Figs. 1i-1k). The first portion 'C' and the second portion 'D' may be of a predefined thickness. The thickness of the first portion 'C' may be in the range of 0.10-1.20mm, more preferably 0.45- 0.65mm and the thickness of the second portion 'D' may be in the range of 0.05- 3.5mm, more preferably 1.0- 2.0mm. The thickness of the one or more stopper 9 plays an important role to maintain the required flexibility of the one or more stoppers 9. The one or more stoppers 9 having a high thickness will have less flexibility and if the thickness is very low, the chances of breakage of the one or more stopper 9 becomes high. Therefore, the one or more stoppers 9 of the present invention include an intermediate value of thickness to overcome the aforesaid problems.

As depicted in Figs. 1i-1k, the first portion 'C' of the one or more stoppers 9 is conical in shape and includes a lower end (marked as 'C1' Figs. 1i-1k) and an upper end (marked as 'C2' Figs. 1i-1k).

In an embodiment, the lower end 'C1' includes a uniform periphery as depicted in Figs. 1i-1k. The lower end 'C1' may be permanently and/or temporarily attached to the second portion 'D'. In an embodiment, the lower end 'C1' includes predefined inner and outer diameters. The outer diameter of lower end 'C1' may be in a range of 1.5mm to 6.5mm, more preferably 3.5mm to 4.5mm. The inner diameter of lower end 'C1' may be in a range of 0.5mm to 5.0mm, more preferably 2.0mm to 3.5mm.

In an embodiment, the upper end 'C2' is a free end. The upper end 'C2' may be of various shapes as depicted in Figs. 1i-1k. In an embodiment, as depicted in Fig. 1i, the upper end 'C2' is a rim-shaped structure having an extendable portion (marked as 'E' in Fig. 1i). The said structure is highly efficient when high push force is required during the deployment procedure due to narrow arteries of a patient. The said structure also helps to maintain the crimped position of the prosthetic heart valve 10 during the deployment procedure and navigate the delivery catheter 100 through an introducer sheath (not shown). The said structure also helps in uniform expansion of the crimped prosthetic heart valve 10. Fig. 1g depicts a perspective view of the one or more stoppers 9 with the rim-shaped structure having an extendable portion E provided inside the balloon 5.

In another embodiment, as depicted below in Fig. 1j, the upper end C2 is a crown-shaped structure having alternate peaks and valleys. The said structure provides greater flexibility during the expansion/deflation of the balloon 5. The said structure also allows the entry of the additional inflation fluid(s) inside the balloon 5 to expand/deflate the balloon 5. Fig. 1h depicts a perspective view of the one or more stoppers 9 with the crown-shaped structure having alternate peaks and valleys provided inside the balloon 5.

In yet another embodiment, as depicted below in Fig. 1k, the upper end C2 includes a uniform periphery. The uniform periphery of the second end C2 helps in reducing the crimping profile of the prosthetic heart valve 10. The said uniform periphery also reduces the overall diameter of the crimped prosthetic heart valve 10 for easy navigation during the deployment process. Fig. 1f depicts a perspective view of the one or more stoppers 9 with the uniform periphery provided inside the balloon 5.

In an embodiment, the upper end 'C2' includes predefined inner and outer diameters. The outer diameter of upper end 'C2' may be in a range of 4.5mm to 9.5mm, more preferably 6.5mm to 7.5mm. The inner diameter of upper end 'C2' may be in a range of 1.5mm to 6.5mm, more preferably 3.5mm to 4.5mm.

In an embodiment, as depicted in Figs. 1i-1k, the second portion 'D' of the one or more stoppers 9 is cylindrical in shape. The second portion 'D' may include a predefined diameter which may be in the range of 3.5mm to 4.5 mm. The second portion 'D' may include a predefined length which may be in the range of 3.3 mm to 5.0 mm. The second portion 'D' may include at least one hole (marked as 'D1' in Figs. 1i-1k). In an embodiment, the at least one hole 'D1' of the second portion 'D' is utilized to attach the one or more stoppers 9 with the support tube 7. The at least one hole 'D1' of the second portion 'D' may be of any shape known in the art. In an embodiment, the at least one hole 'D1' is circular in shape. The diameter of the at least one hole D1 may be in the range of 0.1- 2.0mm, more preferably 0.5- 1.5mm.

In an embodiment, the first portion 'C' and the second portion 'D' when coupled together depict a funnel shape of the one or more stoppers 9 (as depicted in Figs. 1i, 1j and 1k).

The one or more stoppers 9 are utilized to avoid dislodgement of the prosthetic heart valve 10 during the transfer of the said valve from the femoral access to the target location. In an embodiment, the prosthetic heart valve 10 is placed between the one or more stoppers 9. The length of the one or more stoppers 9 may be in the range of 8- 20mm, more preferably 12-16mm. In an embodiment, the length of the one or more stoppers 9 is 16mm.

The handle 11 is provided near the proximal end 'A' of the delivery catheter 100. The handle 11 may include without limitation, a threaded main shaft (not shown). The handle 11 guides the push-pull mechanism or the steerable mechanism of the outer shaft 1. The length of the handle 11 may be in the range of 80- 220mm, more preferably 120- 180mm.

In an embodiment, the handle 11 is provided in the form of a housing inside which the threaded main shaft is provided. The length of the housing of the handle 11 may be in the range of 60- 160mm, more preferably 100- 120mm.

The length of the threaded main shaft of the handle 11 may be in range of 50- 100, more preferably 60- 80mm.

The soft tip 13 is provided at the secondary end 'B' of the delivery catheter 100 and is coupled to the distal end 5b of the balloon 5 (as depicted in Fig. 1c). The soft tip 13 is provided to facilitate the advancement of the delivery catheter 100 during the deployment procedure. The soft tip 13 further helps in guiding the delivery catheter 100 through the tortuous vessels without puncturing or damaging a vessel as it flexes through the vascular system of the patient during the deployment procedure.

The soft tip 13 may be made of without limitation pebax, polymer, polyurethane etc. In an embodiment, the soft tip 13 is made of low durometer polymer. In an embodiment, the soft tip 13 is conical in shape. The soft tip 13 may have an outer diameter in the range of 4-8mm. The soft tip 13 may have a total length in the range of 10-20mm.

Optionally/additionally, the delivery catheter 100 may include an introducer sheath (not shown). The introducer sheath is used to deploy the crimped prosthetic heart valve 10 at the target location.

Fig. 2 illustrates the deployment procedure of the prosthetic heart valve 10 at the target location with the help of the delivery catheter 100. The deployment procedure commences at step 201.

At step 201, the delivery catheter 100 is advanced into a patient's vasculature. The said step is performed in order to deploy the prosthetic heart valve 10 in the diseased native valve of the patient (i.e. the target location). In an embodiment, the prosthetic heart valve 10 along with the balloon 5 is deployed in a crimped state. The said step is performed by utilizing the steerable mechanism having the pull and release wire mechanism of the outer shaft 1.

When the delivery catheter enters inside the patient's vasculature, the soft tip 13 of the delivery catheter 100 acts as a guide for the advancement for the delivery catheter 100. After entering the patient's vasculature, the delivery catheter 100 passes through various curvatures in the patient's arteries. In order to pass smoothly through the said curvatures, the pull wire 1c which is coupled with a puller (not shown) pulls the said wire 1c in a direction of the aortic arc. The puller is combined with the threaded shaft of the handle 11 in order to bend the secondary end 'B' of the delivery catheter 100. The threaded main shaft is rotated (say, clockwise) which in turn moves the puller towards the end of the handle 11. The said movement stretches the pull wire ring and the flexible part of the outer shaft 1 bends accordingly. The aforesaid step is performed till the delivery catheter 100 reaches the native valve of the patient (i.e. the target location).

At step 203, the crimped balloon 5 (which is provided on the delivery catheter 100) is expanded to cause the crimped prosthetic heart valve 10 to adhere to the native valve annulus. Post entering the target location, the inflation fluid enters the balloon 5 through the support tube 7 and the one or more stoppers 9.

Firstly, the inflation fluid(s) expands the proximal end 5a and the distal end 5b of the balloon 5. The expansion of the distal end 5b and the proximal end 5a of the balloon 5 helps to maintain the position of the crimped prosthetic heart valve 10. The aforesaid expansion also helps in maintaining the expansion height of the crimped prosthetic heart valve 10.

The inflation fluid(s) then enters the middle portion 5c of the balloon 5. Due to accumulation of the inflation fluid(s) inside the balloon 5, the balloon 5 gradually expands at the circumferential region without hampering the crimped profile of the prosthetic heart valve 10. This results in uniform expansion of the prosthetic heart valve 10 at the target location.

Post the expansion of the prosthetic heart valve 10, the inflation fluid is withdrawn via the hub 1d of the outer shaft 1 leaving the balloon 5 deflated.

At step 205, the delivery catheter 100 is retracted from the patient's vasculature. The retraction step is performed with the help of the steerable mechanism having the pull and release wire mechanism of the outer shaft 1 (as described in step 201) without hampering the vessels inside the patient's vasculature.

The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A prosthetic heart valve assembly comprising:
a. a prosthetic heart valve (10) mounted over a balloon (5) for deployment at a target location, wherein dimensions of the prosthetic heart valve (10) mimic the anatomy of a native heart valve;
b. a delivery catheter (100) for deployment of the prosthetic heart valve (10), wherein the delivery catheter (100) including:
i. an outer shaft (1) including an outer first end (1a), an outer second end (1b) and a lumen, the outer first end (1a) having a diameter greater than the outer second end (1b), wherein the outer shaft (1) includes one or more layers;
ii. an inner shaft (3) provided inside the lumen of the outer shaft (1) including an inner first end (3a) and an inner second end (3b), wherein the inner shaft (3) includes one or more layers;
iii. a support tube (7) disposed on top of the inner second end (3b) of the inner shaft (3), wherein the support tube (7) includes one or more holes (7a) for passing an inflation fluid, the support tube (7) is provided with the balloon (5) on its outer surface;
iv. a handle (11) provided at an end of the delivery catheter (100); and
v. one or more stoppers (9) mounted on an outer surface of the support tube (7) and within the balloon (5), the one or more stoppers (9) having a first portion (C) and a second portion (D).

2. The prosthetic heart valve assembly as claimed in claim 1 wherein, the outer shaft (1) includes at least three layers, the three layers including an inner layer, a middle layer and an outer layer, wherein the middle layer is braided over the inner layer.

3. The prosthetic heart valve assembly as claimed in claim 1 wherein, the outer shaft (1) includes a hub (1d) utilized to pass an inflation fluid to expand the balloon 5.

4. The prosthetic heart valve assembly as claimed in claim 1 wherein, the outer shaft (1) includes a steerable mechanism for deploying the prosthetic heart valve (10).

5. The prosthetic heart valve assembly as claimed in claim 1 wherein, the inner shaft (3) includes at least three layers, the three layers including a first layer, an intermediate layer and a second layer, wherein the intermediate layer includes a braided configuration.

6. The prosthetic heart valve assembly as claimed in claim 1 wherein, the first portion (C) having a conical shape includes a lower end (C1) and an upper end (C2).

7. The prosthetic heart valve assembly as claimed in claim 6 wherein, the lower end (C1) includes a uniform periphery and the upper end (C2) has a rim-shaped structure with an extendable portion (E).

8. The prosthetic heart valve assembly as claimed in claim 6 wherein, the lower end (C1) includes a uniform periphery and the upper end (C2) has a crown-shaped structure including alternate peaks and valleys.

9. The prosthetic heart valve assembly as claimed in claim 1 wherein, the second portion (D) having a cylindrical shape includes at least one hole (D1), the at least one hole (D1) is utilized to attach the one or more stoppers (9) with the support tube (7).

10. The prosthetic heart valve assembly as claimed in claim 1 wherein, the dimensions of the prosthetic heart valve (10) include a diameter ranging from 18mm to 34mm.

11. The prosthetic heart valve assembly as claimed in claim 1 wherein, the dimensions of the prosthetic heart valve (10) include a height ranging from 14mm to 28mm.

## Patentansprüche

1. Herzklappenprothesenanordnung, umfassend:
a. eine Herzklappenprothese (10), die über einem Ballon (5) zur Entfaltung an einer Zielstelle montiert ist, wobei die Abmessungen der Herzklappenprothese (10) die Anatomie einer nativen Herzklappe nachahmen;
b. einen Abgabekatheter (100) zur Entfaltung der Herzklappenprothese (10), wobei der Abgabekatheter (100) Folgendes beinhaltet:
i. einen äußeren Schaft (1), der ein äußeres erstes Ende (1a), ein äußeres zweites Ende (1b) und ein Lumen beinhaltet, wobei das äußere erste Ende (1a) einen Durchmesser aufweist, der größer als das äußere zweite Ende (1b) ist, wobei der äußere Schaft (1) eine oder mehrere Schichten beinhaltet;
ii. einen inneren Schaft (3), der im Inneren des Lumens des äußeren Schafts (1) bereitgestellt ist und ein inneres erstes Ende (3a) und ein inneres zweites Ende (3b) beinhaltet, wobei der innere Schaft (3) eine oder mehrere Schichten beinhaltet;
iii. ein Stützrohr (7), das oben auf dem inneren zweiten Ende (3b) des inneren Schafts (3) angeordnet ist, wobei das Stützrohr (7) ein oder mehrere Löcher (7a) zum Durchleiten eines Aufblasfluids beinhaltet, wobei das Stützrohr (7) an seiner äußeren Fläche mit dem Ballon (5) versehen ist,
iv. einen Griff (11), der an einem Ende des Abgabekatheters (100) bereitgestellt ist; und
v. einen oder mehrere Stopper (9), die an einer äußeren Fläche des Stützrohrs (7) und innerhalb des Ballons (5) montiert sind, wobei der eine oder die mehreren Stopper (9) einen ersten Abschnitt (C) und einen zweiten Abschnitt (D) aufweisen.

2. Herzklappenprothesenanordnung nach Anspruch 1, wobei der äußere Schaft (1) mindestens drei Schichten beinhaltet, wobei die drei Schichten eine innere Schicht, eine mittlere Schicht und eine äußere Schicht beinhalten, wobei die mittlere Schicht über die innere Schicht geflochten ist.

3. Herzklappenprothesenanordnung nach Anspruch 1, wobei der äußere Schaft (1) eine Nabe (1d) beinhaltet, die verwendet wird, um ein Aufblasfluid durchzuleiten, um den Ballon 5 auszudehnen.

4. Herzklappenprothesenanordnung nach Anspruch 1, wobei der äußere Schaft (1) einen lenkbaren Mechanismus zum Entfalten der Herzklappenprothese (10) beinhaltet.

5. Herzklappenprothesenanordnung nach Anspruch 1, wobei der innere Schaft (3) mindestens drei Schichten beinhaltet, wobei die drei Schichten eine erste Schicht, eine Zwischenschicht und eine zweite Schicht beinhalten, wobei die Zwischenschicht eine geflochtene Konfiguration beinhaltet.

6. Herzklappenprothesenanordnung nach Anspruch 1, wobei der erste Abschnitt (C), der eine konische Form aufweist, ein unteres Ende (C1) und ein oberes Ende (C2) beinhaltet.

7. Herzklappenprothesenanordnung nach Anspruch 6, wobei das untere Ende (C1) einen einheitlichen Umfang beinhaltet und das obere Ende (C2) eine randförmige Struktur mit einem ausdehnbaren Abschnitt (E) aufweist.

8. Herzklappenprothesenanordnung nach Anspruch 6, wobei das untere Ende (C1) einen einheitlichen Umfang beinhaltet und das obere Ende (C2) eine kronenförmige Struktur aufweist, die abwechselnde Spitzen und Täler beinhaltet.

9. Herzklappenprothesenanordnung nach Anspruch 1, wobei der zweite Abschnitt (D), der eine zylindrische Form aufweist, mindestens ein Loch (D1) beinhaltet, wobei das mindestens eine Loch (D1) verwendet wird, um den einen oder die mehreren Stopper (9) mit dem Stützrohr (7) zu befestigen.

10. Herzklappenprothesenanordnung nach Anspruch 1, wobei die Abmessungen der Herzklappenprothese (10) einen Durchmesser im Bereich von 18 mm bis 34 mm beinhalten.

11. Herzklappenprothesenanordnung nach Anspruch 1, wobei die Abmessungen der Herzklappenprothese (10) eine Höhe im Bereich von 14 mm bis 28 mm beinhalten.

## Revendications

1. Ensemble valvule cardiaque prothétique comprenant :
a. une valvule cardiaque prothétique (10) montée sur un ballonnet (5) permettant un déploiement au niveau d'un emplacement cible, les dimensions de la valvule cardiaque prothétique (10) reproduisant l'anatomie d'une valvule cardiaque native ;
b. un cathéter de pose (100) permettant le déploiement de la valvule cardiaque prothétique (10), ledit cathéter de pose (100) comprenant :
i. une tige externe (1) comprenant une première extrémité externe (1a), une seconde extrémité externe (1b) et une lumière, la première extrémité externe (1a) possédant un diamètre supérieur à la seconde extrémité externe (1b), ladite tige externe (1) comprenant une ou plusieurs couches ;
ii. une tige interne (3) pourvue à l'intérieur de la lumière de la tige externe (1) comprenant une première extrémité interne (3a) et une seconde extrémité interne (3b), ladite tige interne (3) comprenant une ou plusieurs couches ;
iii. un tube support (7) disposé sur la seconde extrémité interne (3b) de la tige interne (3), ledit tube support (7) comprenant un ou plusieurs trous (7a) pour le passage d'un fluide de gonflage, ledit tube support (7) étant pourvu avec le ballonnet (5) sur sa surface externe ;
iv. une poignée (11) pourvue au niveau d'une extrémité du cathéter de pose (100) ; et
v. une ou plusieurs butées (9) montées sur une surface externe du tube support (7) et à l'intérieur du ballonnet (5), lesdites une ou plusieurs butées (9) possédant une première partie (C) et une seconde partie (D).

2. Ensemble valvule cardiaque prothétique selon la revendication 1, ladite tige externe (1) comprenant au moins trois couches, lesdites trois couches comprenant une couche interne, une couche intermédiaire et une couche externe, ladite couche intermédiaire étant tressée sur la couche interne.

3. Ensemble valvule cardiaque prothétique selon la revendication 1, ladite tige externe (1) comprenant un moyeu (1d) utilisé pour faire passer un fluide de gonflage afin de dilater le ballonnet (5).

4. Ensemble valvule cardiaque prothétique selon la revendication 1, ladite tige externe (1) comprenant un mécanisme orientable permettant de déployer ladite valvule cardiaque prothétique (10).

5. Ensemble valvule cardiaque prothétique selon la revendication 1, ladite tige interne (3) comprenant au moins trois couches, lesdites trois couches comprenant une première couche, une couche intermédiaire et une seconde couche, ladite couche intermédiaire comprenant une configuration tressée.

6. Ensemble valvule cardiaque prothétique selon la revendication 1, ladite première partie (C) présentant une forme conique comprenant une extrémité inférieure (C1) et une extrémité supérieure (C2).

7. Ensemble valvule cardiaque prothétique selon la revendication 6, ladite extrémité inférieure (C1) comprenant une périphérie uniforme et ladite extrémité supérieure (C2) présentant une structure en forme de rebord avec une partie extensible (E).

8. Ensemble valvule cardiaque prothétique selon la revendication 6, ladite extrémité inférieure (C1) comprenant une périphérie uniforme et ladite extrémité supérieure (C2) présentant une structure en forme de couronne comprenant des pics et des creux alternés.

9. Ensemble valvule cardiaque prothétique selon la revendication 1, ladite seconde partie (D) présentant une forme cylindrique comprenant au moins un trou (D1), ledit trou (D1) étant utilisé pour fixer ladite ou lesdites butées (9) au tube support (7).

10. Ensemble valvule cardiaque prothétique selon la revendication 1, lesdites dimensions de la valvule cardiaque prothétique (10) comprenant un diamètre allant de 18 mm à 34 mm.

11. Ensemble valvule cardiaque prothétique selon la revendication 1, lesdites dimensions de la valvule cardiaque prothétique (10) comprenant une hauteur allant de 14 mm à 28 mm.
